# EUROPÄISCHE PATENTANMELDUNG

(11) **EP 1 502 911 A1**
(43) Veröffentlichungstag der Anmeldung: **02.02.2005**
(21) Anmeldenummer: 04024142.4
(22) Anmeldetag: 19.12.2001
(51) Int. Cl.: C07C 209/70, C07C 209/60

(54) **Verfahren zur Herstellung von n-Butenylaminen**

(30) Priorität: 07.03.2001 DE 10110842
(62) Teilanmeldung aus: 01130182.7
(71) Anmelder: BASF AKTIENGESELLSCHAFT, 67056 Ludwigshafen (DE)
(72) Erfinder: Funke, Frank, Dr., 67067 Ludwigshafen (DE); Steinbrenner, Ulrich, Dr., 67435 Neustadt (DE); Zehner, Peter, Dr., 67071 Ludwigshafen (DE); Böhling, Ralf, Dr., 64347 Griesheim (DE); Harder, Wolfgang, Dr., 69469 Weinheim (DE)

(57) **Zusammenfassung**

Verfahren zur Herstellung eines 1,3-Butadien-freien Gemischs aus einem entsprechenden 1,3-Butadien-haltigen Gemisch, wobei man 1,3-Butadien in Form eines 1,3-Butadien-haltigen Gemischs, das man bei der Spaltung von Erdölfraktionen oder bei der Dehydrierung von LPG oder LNG oder aus der GTL-Technologie erhält, mit einem primären Amin und/oder einem sekundären Amin unter hydroaminierenden Bedingungen umsetzt, wobei man die Hydroaminierung in Gegenwart eines Alkalimetalls als Katalysator oder die Hydroaminierung in Gegenwart eines Metallmonoalkylamids oder Metalldialkylamids oder eines dem eingesetzten Amin entsprechenden Metallamids als Katalysator durchführt, und anschließend das oder die erhaltene/n Hydroaminierungsprodukt/e in einer zweiten Verfahrensstufe unter umalkylierenden Bedingungen umsetzt.

## Beschreibung

Die vorliegende Erfindung betrifft Verfahren zur Herstellung von n-Butylaminen und den dabei als Zwischenprodukt auftretenden n-Butenylaminen.

n-Butylamine dienen als Ausgangsstoffe für die Herstellung von Tensiden, Textil- und Flotationshilfsmitteln, Bakteriziden, Korrosions- und Schauminhibitoren, Additiven für Pharmazeutika sowie als Antioxidantien für Fette und Öle.

Alkylamine, wie Butylamine, können durch die Hydrierung von entsprechenden Nitrilen oder Nitroverbindungen, durch die reduktive Aminierung von entsprechenden Aldehyden und Ketonen und durch die Aminierung von entsprechenden Alkoholen hergestellt werden.

Die niederen Alkylamine (C₁₋₁₅-Alkylamine), wie z.B. auch Butylamine (z.B. C₄- bis C₁₅-Butylamine, wie n-Butylamin, Butylmethylamine, Butylethylamine, Butylisopropylamine und Dibutylamine), werden technisch insbesondere durch die Aminierung des korrespondierenden Alkohols oder der korrespondierenden Carbonylverbindung mit Ammoniak, einen primären oder sekundären Amin an Metallkatalysatoren, die z.B. geträgert sind, unter hydrierenden Bedingungen hergestellt (siehe z.B.: Ullmann's Encyclopedia of Industrial Chemistry, Vol. A 2, 5^{th} Ed., Seite 4).

Alternativ können Alkylamine, z.B. Butylamine, auch an sauren Phosphatkatalysatoren aus entsprechenden Alkoholen hergestellt werden (siehe z.B. US-A-4,582,904 (Air Products)).

Eine weitere Alternative zur Herstellung von Alkylaminen, wie z.B. Butylaminen, besteht in der Addition von NH₃ oder Aminen an Olefine in Gegenwart von sauren Katalysatoren, wie z.B. Zeolithen (siehe z. B. EP-A-132 736), in Gegenwart von basischen Katalysatoren, wie z.B. Metallamiden, insbesondere Alkali- und Erdalkalimetallamiden, (siehe z.B. B.W. Howk et al., J. Am. Chem. Soc. 76, Seite 1899ff (1954); R. Stroh et al., Angew. Chem. 69, 124ff (1957)), Amiden der IV. Nebengruppe (siehe z.B. D. Steinborn et al. (Z. Chem. 29 (1989), Seite 333ff) oder Alkalialkoholaten, oder in Gegenwart von Übergangsmetallkomplexverbindungen (siehe z.B. US-A-3,758,586).

EP-A-752 409 (DE-A-195 24 240) beschreibt ein Verfahren zur Herstellung von Aminen durch Hydroaminierung von Olefin-haltigen Gemischen, die man bei der Spaltung von Erdölfraktionen erhält, an Zeolithen, Alumosilikaten, Phosphaten, mesoporösen Oxiden, Pillard Clays, amorphen Oxiden oder Schichtsilikaten. Insbesondere werden hier Butadien-freie C4-Kohlenwasserstoffgemische eingesetzt (Seite 3, Zeilen 10-12; Beispiele).

Die oben angeführten Verfahren zur Herstellung von Alkylaminen weisen folgende Nachteile auf:

Der Einsatz von Alkoholen (z.B. Ethanol), Aldehyden, Ketonen und Nitrilen als Ausgangsstoffe (Edukte) für die Herstellung von Alkylaminen, wie Butylaminen, ist bezogen auf deren Preise wesentlich unwirtschaftlicher als der Einsatz von entsprechenden Olefinen (z.B. Ethen).

Der Einsatz von Olefinen als Ausgangsstoff für die Alkylaminherstellung ist demnach wünschenswert, jedoch bisher mit folgenden Nachteilen behaftet (vgl. z.B.: M. Beller et al., Chem. Rev. 98, 675f (1998) 675; R. Taube, ,Reaction with Nitrogen Compounds' in B. Cornils und W. A. Hermann: ,Applied Homogeneous Catalysis with Organometallic Compounds', VCH Weinheim, 1996, Seiten 507 bis 520, und E. Haak et al., Chemie in unserer Zeit (1999), 297 bis 303, insbesondere die Zusammenfassung auf S. 302):
- aa): Die basisch heterogen katalysierte Addition von Aminen an Olefine in Gegenwart von Metalloxid-Katalysatoren gelingt nach Kakuno et al., J. Catal. 85 (1984), Seite 509ff, mit primären und sekundären Alkylaminen und konjugierten Dienen wie Butadien oder Isopren.
- ab): Die schwach basisch katalysierte Addition von Aminen an Olefine mit Alkalialkoholat als Katalysator ist nach Beller et al., Angew. Chem. 110 (1998), Seite 3571ff, nur erfolgreich im Falle von aromatisch konjugierten Aminen und Styrol als Olefinkomponente.
- ac): Bei der NaNH₂- oder KNH₂-katalysierten Addition von NH₃ an Olefine, wie sie z.B. in B.W. Howk et al., J. Am. Chem. Soc. 76 (1954), 1899-1902 und R.D. Closson et al., US-A-2,750,417 beschrieben ist, sind die Raum-Zeit-Ausbeuten an gewünschten Alkylaminen selbst bei hohen Temperaturen und Olefindrucken aufgrund der geringen Aktivität und Löslichkeit des Metallamids sehr klein.
- ad): G.P. Pez (US-A-4,336,162 und US-A-4,302,603) beschreibt einen Lösungsansatz für das in ac) genannte Problem durch den Wechsel zu den entsprechenden Rb- und Cs-Amiden oder die Verwendung eines Eutektikums von NaNH₂ und KNH₂. Im ersten Fall verbietet sich die technische Realisierung wegen des extrem hohen Katalysatorpreises, im zweiten Fall sind die Raum-Zeit-Ausbeuten an gewünschten Alkylaminen immer noch zu klein.
- b): An sauren Katalysatoren, wie z.B. Zeolithen, gelingt die Addition von NH₃ an Olefine, wobei Ammoniak in der Regel in hohem Überschuss bezogen auf das Olefin eingesetzt wird, nicht in jedem Fall mit so guten Selektivitäten und Ausbeuten für ein bestimmtes Alkylamin wie z.B. im Fall von Isobuten (siehe z.B. DE-A-36 34 247).
Die Hydroaminierung von Olefinen mit sekundären Aminen in Gegenwart saurer Katalysatoren verläuft wiederum im allgemeinen in schlechteren Ausbeuten und mit schlechteren Selektivitäten als die entsprechende Hydroaminierung mit Ammoniak oder primären Aminen.
- c): Die Übergangsmetallkomplex-katalysierte Hydroaminierung von Olefinen gelingt im allgemeinen nur mit sekundären Alkylaminen in guten Ausbeuten (z.B.: Brunet, Gazzetta Chimica Italiana, 127, 1997, Seiten 111 bis 118, Seite 112, linke Spalte) .

Die beiden älteren deutschen Anmeldungen Nr. 10030619.5 vom 28.06.00 und Nr. 10041676.4 vom 24.08.00 beschreiben ein Verfahren zur Herstellung von Alkylaminen, wobei man in einer ersten Verfahrensstufe ein Olefin mit Ammoniak, einem primären Amin und/oder einem sekundären Amin unter hydroaminierenden Bedingungen umsetzt und anschließend das oder die erhaltene/n Hydroaminierungsprodukt/e in einer zweiten Verfahrensstufe unter umalkylierenden Bedingungen umsetzt. Der Einsatz von 1,3-Butadien in Form eines 1,3-Butadien-haltigen Gemischs, das man bei der Spaltung von Erdölfraktionen oder bei der Dehydrierung von LPG oder LNG oder aus der GTL-Technologie erhält, als Olefinkomponente ist hier nicht offenbart.

Nachteilig bei vielen Verfahren zur Herstellung von Aminen aus Olefinen ist, dass die Isolierung von reinen Olefinen oder Mischungen von reinen Olefinen aus Gemischen, die man bei der Spaltung von Erdölfraktionen erhält, aufwendig und teuer ist.

Die aufwendige Abtrennung von 1,3-Butadien aus C4-Schnitt ist z.B. in K. Weissermel und H.-J. Arpe, Industrielle Organische Chemie, 4. Aufl. 1994, VCH Verlagsgesellschaft, Seite 76, beschrieben.

Der vorliegenden Erfindung lag die Aufgabe zugrunde, unter Überwindung der Nachteile des Stands der Technik ein verbessertes, wirtschaftliches und flexibles Verfahren zur Herstellung von n-Butylaminen aufzufinden, das es ermöglicht, ein gewünschtes n-Butylamin oder mehrere gewünschte n-Butylamine mit hoher Raum-Zeit-Ausbeute und Selektivität herzustellen.

Demgemäß wurde ein Verfahren zur Herstellung von n-Butylaminen gefunden, welches dadurch gekennzeichnet ist, dass man zunächst n-Butenylamine durch Umsetzung von 1,3-Butadien in Form eines 1,3-Butadien-haltigen Gemischs, das man bei der Spaltung von Erdölfraktionen oder bei der Dehydrierung von LPG oder LNG oder aus der GTL-Technologie erhält, mit einem primären Amin und/oder einem sekundären Amin unter hydroaminierenden Bedingungen herstellt und das oder die erhaltene/n Butenylamin/e anschließend in einer zweiten Verfahrensstufe unter hydrierenden und umalkylierenden Bedingungen umsetzt.

Die 1,3-Butadien-haltigen Gemische stammen vorzugsweise aus den folgenden Quellen:
- a): C4-Schnitte, die großtechnisch bei der thermischen oder katalytischen Spaltung von Erdölfraktionen, wie z.B. Benzinen, insbesondere Naphtha, anfallen (Steamcracken; siehe z.B. K. Weissermel und H.-J. Arpe, Industrielle Organische Chemie, 4. Aufl. 1994, VCH Verlagsgesellschaft, Seiten 70-76);
- b): Kohlenwasserstoffströme aus der Dehydrierung von sogenanntem LPG oder LNG, vorzugsweise C4-Schnitten daraus, wobei der C₄-Anteil des LPG-Stroms vor oder nach der Dehydrierung vom LPG-Strom abgetrennt wird. Ein LNG-Strom mit hohem C1 und C2 kann dabei z.B. über ein MTO-Verfahren zuvor in C4 überführt werden.

LPG bedeutet dabei Liquified Petroleum Gas (Flüssiggase). Derartige Flüssiggase sind beispielsweise in der DIN 51 622 definiert. Sie enthalten im allgemeinen die Kohlenwasserstoffe Propan, Propen, Butan, Buten und deren Gemische, die in Ölraffinerien als Nebenprodukte bei Destillation und Cracken von Erdöl sowie in der Erdgas-Aufbereitung bei der Benzinabscheidung anfallen. LNG bedeutet Liquified Natural Gas (Erdgas). Erdgas besteht hauptsächlich aus gesättigten Kohlenwasserstoffen, die je nach ihrer Herkunft unterschiedliche Zusammensetzungen aufweisen und im allgemeinen in drei Gruppen eingeteilt werden. Erdgas aus reinen Erdgas-Lagerstätten besteht aus Methan und wenig Ethan. Erdgas aus Erdöl-Lagerstätten enthält zusätzlich noch größere Mengen höher molekularer Kohlenwasserstoffe wie Ethan, Propan, Isobutan, Butan, Hexan, Heptan und Nebenprodukte. Erdgas aus Kondensat- und Destillat-Lagerstätten enthält nicht nur Methan und Ethan, sondern auch in erheblichem Umfang höher siedende Komponenten mit mehr als 7 Kohlenstoffatomen. Für eine nähere Beschreibung von Flüssiggasen und Erdgas kann auf die entsprechenden Stichworte in Römpp, Chemielexikon, 9. Auflage verwiesen werden.

Das als Feedstock verwendete LPG und LNG umfaßt insbesondere sogenannte Feldbutane, wie man die C4-Fraktion der "feuchten" Anteile des Erdgases sowie der Erdölbegleitgase nennt, die durch Trocknung und Abkühlung auf etwa -30°C in flüssiger Form aus den Gasen abgetrennt werden. Duch Tieftemperatur- oder Druckdestillation gewinnt man daraus die Feldbutane, deren Zusammensetzung je nach Lagerstätte schwankt, die jedoch im allgemeinen etwa 30% iso-Butan und etwa 65 % n-Butan enthalten.

Geeignete Verfahren zur Dehydrierung von Kohlenwasserstoffen sind beispielsweise in der DE-A-100 47 642 beschrieben. Weiterhin kann für die Dehydrierung von Alkanen auf US 4,788,371, WO 94/29021, US 5,733,518, EP-A-0 838 534, WO 96/33151 oder WO 96/33150 verwiesen werden.

Das oben erwähnte MTO steht dabei für Methanol-To-Olefin. Es ist mit dem MTG-Verfahren (Methanol-To-Gasoline) verwandt. Es handelt sich dabei um ein Verfahren zur Dehydratisierung von Methanol an ein geeigneten Katalysatoren, wobei ein olefinisches Kohlenwasserstoffgemisch entsteht. Selbst C₁-Feedströme können damit beispielsweise über Methanol und das MTO-Verfahren in Olefin-Gemische überführt werden, aus denen die C₄-Anteile durch geeignete Methoden abgetrennt werden können. Die Abtrennung kann beispielsweise durch Destillation erfolgen. Für das MTO-Verfahren kann auf Weissermel, Arpe, Industrielle organische Chemie, 4. Auflage 1994, VCH-Verlagsgesellschaft, Weinheim, S. 36 ff. verwiesen werden.

Das Methanol-To-Olefin-Verfahren ist zudem beispielsweise in P.J.Jackson, N.White, Technologies for the conversion of natural gas, Austr. Inst. Energy Conference 1985 beschrieben.
c) C4-Schnitte aus der sogenannten GTL-Technologie (GTL = gas to liquid), beispielsweise der Fischer-Tropsch-Synthese.
d) Alle Mischungen aus den vorgenannten Kohlenwasserstoffströmen.

Die bevorzugten C4-Schnitte besitzen folgende Zusammensetzung (in Gew.-%):

### 1) C4-Crackerschnitt:

1,3-Butadien:
   25 - 60, bevorzugt 35 - 50, besonders bevorzugt 40 - 45;
Butene (iso-Buten, 1-Buten, trans-2-Buten, cis-2-Buten):
   35 - 70, bevorzugt 40 - 60;
Butane (n-Butan, iso-Butan):
   3 - 12, bevorzugt 5 - 10;
Alkine (Vinylacetylen, 1-Butin):
   0 - 4, bevorzugt 0 - <2, besonders bevorzugt 0 - <1;
Sonstige (z.B. 1,2-Butadien, C5-Komponenten wie Pentane):
   0 - 2, bevorzugt 0 - 1.

Eine typische C4-Crackerschnitt-Zusammensetzung (in Gew.-%) ist z.B.:

| | |
|---|---|
| 1,3-Butadien | 42,3 |
| Butene | 47,5 |
| Butane | 8,7 |
| Alkine | 0,8 |
| Sonstige | 0,7 |

### 2) Kohlenwasserstoffgemisch aus der Dehydrierung von n-Butan:

1,3-Butadien:
   3 - 40, bevorzugt 5 - 30, besonders bevorzugt 7 - 15;
Butene (1-Buten, trans-2-Buten, cis-2-Buten):
   25 - 70, bevorzugt 35 - 60;
n-Butan:
   25 - 70, bevorzugt 35 - 60;
Alkine (Vinylacetylen, 1-Butin):
   0 - 1, bevorzugt 0 - 0,5, besonders bevorzugt 0 - 0,2;
Sonstige (z.B. 1,2-Butadien):
   0 - 1, bevorzugt 0 - 0,5.

Eine typische Zusammensetzung (in Gew.-%) von C4-Schnitt aus einer n-Butandehydrierung ist z.B.:

| | |
|---|---|
| 1,3-Butadien | 8 |
| Butene | 46 |
| Butane | 45 |
| sonstige | ≤1 |

### 3) Kohlenwasserstoffgemisch aus der Dehydrierung von Feldbutanen:

1,3-Butadien:
   1 - 20, bevorzugt 2 - 15, besonders bevorzugt 3 - 10;
Butene (1-Buten, trans-2-Buten, cis-2-Buten, i-Buten):
   45 - 85, bevorzugt 55 - 80;
Butane (n-Butan, i-Butan):
   10 - 50, bevorzugt 15 - 40;
Sonstige:
   0 - 1, bevorzugt 0 - 0,5.

Eine typische Zusammensetzung (in Gew.-%) von C4-Schnitt aus einer n-Butandehydrierung ist z.B.:

| | |
|---|---|
| 1,3-Butadien | 6 |
| Butene | 66 |
| Butane | 27 |
| sonstige | 1 |

Besonders bevorzugt wird ein C4-Schnitt eingesetzt, der weniger als 2 Gew.-%, insbesondere weniger als 1 Gew.-%, Kohlenwasserstoffe enthält, die nach der MSAD-Skala einen pKs-Wert kleiner 30 besitzen.

Bei solchen Kohlenwasserstoffen handelt es sich z.B. um 1-Butin und Vinylacetylen.

[Definition der MSAD-Skala ("Mc Ewen-Streitwieser-Applequist-Dessy-Skala") z.B. in D.J. Cram, Fundamentals of Carbanion Chemistry, Acad. press, 1965].

Überraschenderweise gelingt es mit den erfindungsgemäßen Verfahren, dass bei der Umsetzung des primären oder sekundären Amins mit dem 1,3-Butadien-haltigen und weitere Olefine enthaltenden Gemisch das Amin mit einer Olefin-Selektivität, bezogen auf alle Olefine im Gemisch, von größer 90 %, insbesondere größer 95 %, ganz besonders größer 98 %, mit dem 1,3-Butadien zum entsprechenden n-Butenylamin als Hydroaminierungsprodukt reagiert. Aus dem 1,3-Butadien-haltigen Gemisch reagiert also praktisch ausschließlich das 1,3-Butadien ab.

Besonders bevorzugt werden die Reaktionsbedingungen (siehe unten) so gewählt, dass das 1,3-Butadien vollständig (d.h. zu größer 98 %, insbesondere größer 99 %) aus dem 1,3-Butadien-haltigen Gemisch entfernt wird.

Die nach Durchführung der Umsetzung verbleibenden Bestandteile des eingesetzten 1,3-Butadien-haltigen Gemischs können nach der Abtrennung und gegebenenfalls Reinigung weiterverarbeitet und anderweitig verwendet werden. Zum Beispiel können diese verbleibenden Bestandteile wieder in einem Steamcracker zurückgeleitet werden.

Enthalten die nach Durchführung der Umsetzung verbleibenden Bestandteile des eingesetzten 1,3-Butadien-haltigen Gemischs nach geringe Mengen an 1,3-Butadien, können diese Bestandteile in eine 1,3-Butadien-Partialhydrierungsstufe zurückgeführt werden (K. Weissermel und H.-J. Arpe, Industrielle Organische Chemie, 4. Aufl. 1994, VCH Verlagsgesellschaft, Seite 76, 2. Absatz).

Insbesondere können die nach Durchführung der Umsetzung verbleibenden Bestandteile des eingesetzten 1,3-Butadien-haltigen Gemischs bei durch die Umsetzung erfolgter vollständiger Entfernung des 1,3-Butadiens nach ihrer Abtrennung als Raffinat I weiterverarbeitet werden.

Ein solches erfindungsgemäß erhaltenes Raffinat I besteht aus n-Butan, iso-Butan, 1-Buten, cis-2-Buten, trans-2-Buten, iso-Buten, weniger als 1 Gew.-%, insbesondere weniger als 0,5 Gew.-%, ganz besonders 0-0,1 Gew.-% 1,3-Butadien, 0-0,1 Gew.-% Alkinen und gegebenenfalls einem weiteren Rest (z.B. Spuren an C5-Komponenten).

Vor der Weiterverarbeitung der nach Durchführung der Umsetzung verbleibenden Bestandteile des eingesetzten 1,3-Butadien-haltigen Gemischs kann eine Reinigung dieser Bestandteile durch Behandlung mit einem sauren Ionenaustauscher, einem sauren Heterogenkatalysator (z.B. Zeolith, saures Mischoxid, γ-Aluminiumoxid), mit verdünnter Säure (extraktiv), oder durch Destillation erfolgen. Durch diese Behandlung werden z.B. noch vorhandene basische Komponenten, wie Amin-Verunreinigungen, entfernt.

Insofern ist das erfindungsgemäße Verfahren auch ein Verfahren zur Herstellung eines 1,3-Butadien-freien Gemischs (d.h. 1,3-Butadien-Gehalt kleiner 1 Gew.-%, insbesondere kleiner 0,5 Gew.-%, ganz besonders kleiner 0,1 Gew.-%), z.B. Raffinat I, aus dem entsprechenden 1,3-Butadien-haltigen Gemisch, das bei der Spaltung von Erdölfraktionen oder bei der Dehydrierung von LPG oder LNG oder aus der GTL-Technologie erhalten wurde (z. B. einem C4-Schnitt).

Je nach den gewählten Reaktionsbedingungen (siehe unten) kann es vorkommen, dass auch ein Teil des in dem 1,3-Butadien-haltigen Gemisch enthaltenen 1-Butens mit dem primären und/oder sekundären Amin reagiert. Da dann ebenfalls erwünschte n-Butylamine entstehen, ist diese ,Nebenreaktion' nicht nachteilig.

Im erfindungsgemäßen Verfahren einsetzbare primäre und sekundäre Amine oder Mischungen hiervon sind im allgemeinen Amine der Formel 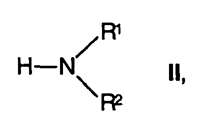 in der
R¹ Wasserstoff (H), Alkyl, insbesondere C₁- bis C₂₀-Alkyl, Alkenyl, insbesondere C₂- bis C₂₀-Alkenyl, Cycloalkyl, insbesondere C₃- bis C₂₀-Cycloalkyl, Alkoxyalkyl, Aminoalkyl, Monoalkylaminoalkyl, Dialkylaminoalkyl, Aryl, Aralkyl
und R² Alkyl, insbesondere C₁- bis C₂₀-Alkyl, Alkenyl, insbesondere C₂- bis C₂₀-Alkenyl, Cycloalkyl, insbesondere C₃- bis C₂₀-Cycloalkyl, Alkoxyalkyl, Aminoalkyl, Monoalkylaminoalkyl, Dialkylaminoalkyl, Aryl, Aralkyl bedeuten und
R¹ und R² gemeinsam eine gesättigte oder ungesättigte C₃- bis C₉-Alkylenkette, die durch ein O-, S- oder N-Heteroatom unterbrochen sein kann, insbesondere gemeinsam eine -(CH₂)ⱼ-X-(CH₂)ₖ-Gruppe, mit j und k = 1 bis 4 und X = CH₂, CHR³, Sauerstoff (O), Schwefel (S) oder NR³, mit R³ = H oder C₁- bis C₄-Alkyl, bedeuten kann.

Beispiele für primäre Amine gemäß Formel II sind Methylamin, Ethylamin, n-Propylamin, iso-Propylamin, n-Butylamin, iso-Butylamin, tert.-Butylamin, n-Pentylamin, Cyclopentylamin, iso-Pentylamin, n-Hexylamin, Cyclohexylamin, n-Octylamin, n-Decylamin, 1-Phenylethylamin, 2-Phenylethylamin, Allylamin, 2-Dimethylaminoethylamin und 2-Methoxyethylamin.

Beispiele für sekundäre Amine gemäß Formel II sind Dimethylamin, Diethylamin, Di-n-propylamin, Diisopropylamin, Di-n-butylamin, Pyrrolidin, Piperidin, Hexamethylenamin, Piperazin und Morpholin.

Als Katalysator bei der Herstellung von n-Butenylaminen durch Umsetzung von 1,3-Butadien in Form eines 1,3-Butadien-haltigen Gemischs, das man bei der Spaltung von Erdölfraktionen erhält, mit einem primären oder sekundären Amin wird ein Alkalimetall, ein Metallmonoalkylamid oder Metalldialkylamid, ein dem eingesetzten Amin entsprechendes Metallamid oder eine Übergangsmetallkomplexverbindung eingesetzt.

Alkalimetalle, insbesondere Na und K, ganz besonders Na, sind als Katalysatoren bevorzugt.

Das Alkalimetall reagiert in situ mit dem primären und/oder sekundären Amin und dem 1,3-Butadien zum entsprechenden Alkalimetallamid.

Bei den Metallamid-Katalysatoren sind Metalldialkylamide gegenüber Metallmonoalkylamiden bevorzugt.

Die Metallmonoalkylamide und Metalldialkylamide besitzen im allgemeinen die Formel MNR⁴R⁵ (M = einwertiges Metall), M(NR⁴R⁵)₂ (M = zweiwertiges Metall), M(NR⁴R⁵)₃ (M = dreiwertiges Metall) oder M(NR⁴R⁵)₄ (M = vierwertiges Metall), wobei

R⁴ die Bedeutungen gemäß dem Rest R¹ und R⁵ die Bedeutungen gemäß dem Rest R² hat und R⁴ und R⁵ auch gemeinsam eine gesättigte oder ungesättigte C₃- bis C₉-Alkylenkette, die durch ein O-, S- oder N-Heteroatom unterbrochen sein kann (wie für R¹ und R² definiert), insbesondere gemeinsam eine -(CH₂)ⱼ-X-(CH₂)ₖ- Gruppe (wie für R¹ und R² definiert), bedeuten kann (siehe oben).

Besonders bevorzugt besitzen die Metallamide die Formel MNR¹R², M(NR¹R²)₂, M(NR¹R²)₃ oder M(NR¹R²)₄, in der demnach die Reste R denjenigen des eingesetzten primären und/oder sekundären Amins entsprechen.

Als Metallkomponente (M) in den Metallmonoalkylamid- und Metalldialkylamid-Katalysatoren sind Metalle der Gruppen IA, IIA, IIIB und IVB (Li, Na, K, Rb, Cs, Mg, Ca, Sr, Ba, Sc, Y, La oder ein Lanthanidelement, Ti, Zr oder Hf), insbesondere Li, Na, Mg, Ca und K, ganz besonders Na und K, bevorzugt.

Beispiele für solche Metallalkylamide sind NaHNEt, NaNEt₂, KHNEt, KNEt₂, LiHNEt, LiNEt₂, LiN(iso-C₃H₇)₂, NaN(iso-C₃H₇)₂, NaN(n-C₄H₉)₂, NaHN(iso-C₃H₇), NaHN(iso-Pentyl), Mg(NEt₂)₂, Ca(NPr₂)₂ und Zr(NEt₂)₄.

Bei der Hydroaminierung von Di-n-butylamin mit dem 1,3-Butadien-haltigen Gemisch sind NaN(n-Bu)₂ und KN(n-Bu)₂, bei der von n-Butylamin mit dem 1,3-Butadien-haltigen Gemisch Na(H)N(n-Bu) und K(H)N(n-Bu) besonders bevorzugt.

Die Herstellung dieser Metallamide erfolgt wie in Houben-Weyl, Methoden der organischen Chemie, 4. Aufl., Band XI/2 (Stickstoffverbindungen II und III), Verlag Thieme, Stuttgart, S. 182ff, in US-A-4,595,779, WO 93/14061, DE-A-21 17 970, DRP 615,468, GB-A-742 790, DE-A-26 13 113, US-A-2,750,417, J. Wollensak, Org. Synth. 43 (1963), Seite 45ff, oder C.A. Brown, J. Am. Chem. Soc. 95(3) (1973), Seite 982ff, beschrieben durch Umsetzung von Metall mit dem entsprechenden Amin in Gegenwart einer ungesättigten Verbindung wie z.B. Butadien, Isopren, Naphthalin, Pyridin oder Styrol, durch Umsetzung eines Metallamids oder -hydrids mit dem entsprechenden Amin oder durch Umsetzung einer Organometallverbindung, wie z.B. n-BuLi, MeLi, PhNa, Et₂Mg oder Et₄Zr, mit dem entsprechenden Amin.

Bei der als Katalysator eingesetzten Übergangsmetallkomplexverbindung handelt es sich im allgemeinen um eine Komplexverbindung eines Metalls der Gruppe IIIB, IVB oder VIII des Periodensystems.

Beispiele hierfür sind die in US-A-3,758,586 beschriebenen Verbindungen des Rhodiums oder Iridiums, wie (Cyclooctadien)₂RhCl₂, RhCl₃, RhBr₃ und IrCl₃, für die Umsetzung von sekundären aliphatischen Aminen,
die in US-A-4,454,321 beschriebenen Verbindungen des Rutheniums und Eisens, wie RuCl₃ * xH₂O, Ru(cyclopentadienyl)₂, [Ru(NH₃)₄(OH)Cl]Cl * 2 H₂O, Fe(CO)₅, Fe₂(CO)₉, H₂Fe(CO)₄, Fe(butadien)(CO)₃, Fe(CO)₅ / Tri-n-butylphosphin, Fe(CO)₅ / Triphenylphosphit, für die Umsetzung von primären oder sekundären Aminen, die im Übersichtsartikel von Th.E. Müller und M. Beller in Chem. Rev. 1998, Vol. 98, No. 2, Seiten 675 bis 703, auf den Seiten 679 bis 680 zitierten Verbindungen des Platins und Palladiums, wie PtX₄²⁻ (X = Cl⁻, Br⁻),
die im o. g. Übersichtsartikel von Th.E. Müller und M. Beller auf den Seiten 681 bis 684 zitierten Verbindungen des Lanthans, Neodymiums, Samariums und Lutetiums, wie Cp*₂Ln-E (Cp* = Pentamethylcyclopentadienyl oder Tetramethylcyclopentadienyl mit Brücke zum zweiten Tetramethylcyclopentadienylligand, Ln = La, Nd, Sm oder Lu, E = H, CH(SiMe₃)₂ oder N(SiMe₃)₂), und
die im o. g. Übersichtsartikel von Th.E. Müller und M. Beller auf den Seiten 684 bis 686 zitierten Verbindungen des Platins, Rutheniums, Hafniums, Zirkoniums, Iridiums und Tantals, wie Pt(PEt₃)₂(H)(NHPh), IrCl(C₂H₄)₂(PEt₃)₂ und Cp₂Zr (H) [N(t-Bu) (SiHMe₂)].

Das Verfahren zur Herstellung der n-Butenylamine lässt sich wie folgt ausführen:
1,3-Butadien (I) in Form eines 1,3-Butadien-haltigen Gemischs, das man bei der Spaltung von Erdölfraktionen oder bei der Dehydrierung von LPG oder LNG oder aus der GTL-Technologie erhält, bevorzugt in Form eines 1,3-Butadien-haltigen Gemischs mit einer Zusammensetzung wie oben beschrieben, wird mit einem primären Amin und/oder einem sekundären Amin unter hydroaminierenden Bedingungen umsetzt (Hydroaminierung).

Die Umsetzung erfolgt im allgemeinen gemäß den folgenden Reaktionsschemata.

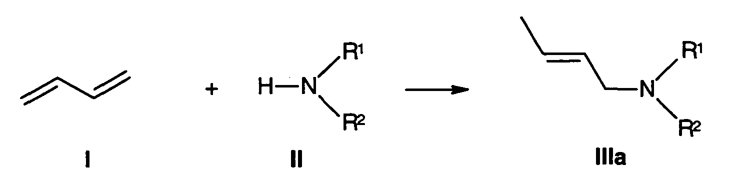

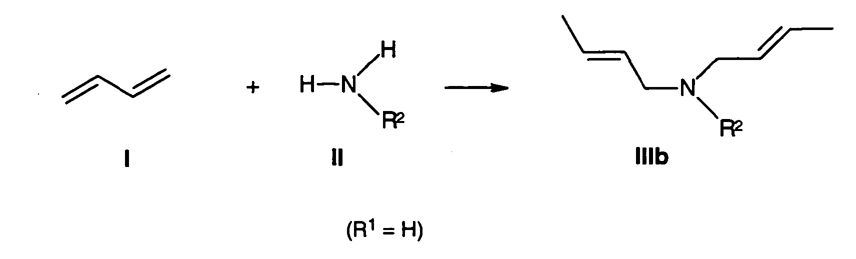

Bei der Umsetzung des 1,3-Butadien-haltigen Gemischs mit einen sekundären Amin resultiert in der Hydroaminierungsstufe ein n-Butenylamin IIIa als tertiäres Amin.

Bei der Umsetzung des 1,3-Butadien-haltigen Gemischs mit einen primären Amin resultiert in der Hydroaminierungsstufe je nach gewählten Reaktionsbedingungen entweder ein n-Butenylamin IIIa als sekundäres Amin (R¹ = H) oder ein Di-n-butenylamin IIIb als tertiäres Amin oder ein Gemisch hiervon.

Vereinfachend für obige Schemata wurde angenommen, dass bei der Hydroaminierung des 1,3-Butadiens die resultierende olefinische Doppelbindung weder in α- noch in β- noch in δ-Position zum Stickstoff in III zu liegen kommt (entsprechend einem Imin bzw. Enamin bzw. Homoallylamin). Unter den basischen Bedingungen kann jedoch in dieser Verfahrensstufe durchaus Doppelbindungsisomerisierung auftreten.

Ganz besonders bevorzugte Amine sind Mono- und Dialkylamine wie Monoethylamin, Diethylamin, n-Butylamin, Di-n-butylamin und Isopropylamin.

Die Hydroaminierungsprodukte ausgehend vom 1,3-Butadien und n-Butylamin sind n-Butenyl-n-butylamin und Di-n-butenyl-n-butylamin, ausgehend vom 1,3-Butadien und Di-n-butylamin Di-n-butyl-n-butenylamin.

### a) Alkalimetall- und Metallamid-Katalyse

Die Umsetzung des 1,3-Butadien-haltigen Gemischs mit dem Amin in Gegenwart des Metallamids (Hydroaminierungsstufe) kann z.B. analog wie in G.P. Pez et al., Pure & Appl. Chem. 57(12), 1917-26 (1985), R.D. Closson et al., J. Org. Chem. 22 (1957), 646-9, G.M. Whitman et al., US-A-2,501,556), D. Steinborn et al., Z. Chem. 29 (1989), 333-4, D. Steinborn et al., Z. Chem. 26 (1986) 349-59 und H. Lehmkuhl et al., J. Organomet. Chem. 55 (1973), 215-20 beschrieben erfolgen.

Die Umsetzung des 1,3-Butadien-haltigen Gemischs mit dem Amin in Gegenwart des Metallamids kann auch in Gegenwart geringer Mengen von Ammoniak (kleiner 1 Mol% bezogen auf das/die eingesetzte/n Amine) durchgeführt werden (vergl. analog DE-A-21 17 970).

Die Herstellung des Metallamids und die katalytische Hydroaminierung des 1,3-Butadien-haltigen Gemischs kann auch in einer "Eintopfsynthese", d.h. simultan durchgeführt werden.

Man setzt dabei eine Organometallverbindung, z.B. BuLi, und das Amin, z.B. Di-n-butylamin, oder (bevorzugt) ein Alkalimetall, z.B. Na, und das Amin, z.B. Di-n-butylamin, ein.

Das Alkalimetall wird hierbei bevorzugt in dispergierter und/oder suspendierter Form eingesetzt, insbesondere in einer Teilchengröße (Durchmesser) von 0,1 bis 1000 µ (Mikrometer). Die Herstellung solcher Alkalimetalldispersionen bzw. -suspensionen erfolgt nach dem Fachmann bekannten Verfahren, z.B. durch entsprechende Einwirkung von Scherkräften, wie z.B. Rühren oder Ultraschall. (Vergl. z.B. ,Organikum', 2. Nachdruck der 15. Auflage, Seite 584, VEB Verlag 1981).

Das Metallamid kann während der Reaktion durch β-Eliminierung oder Einwirkung von H₂ wie in DE-A-26 13 113 beschrieben in Metallhydrid umgewandelt werden; im Falle der β-Eliminierung entsteht dabei parallel ein Imin. Dieses kann unter Einwirkung von primärem oder sekundärem Amin nach DE-A-26 13 113, C.A. Brown, J. Am. Chem. Soc. 95(3) (1973), 982ff oder C.A. Brown, Synthesis (1978), 754ff, wieder in Metallamid und H₂ umgewandelt werden, so dass das Metallhydrid als eine Art "Ruheform" des Metallamids angesehen werden kann und daher im Sinne der vorliegenden Erfindung mit dem Metallamid gleichzusetzen ist.

Weiter können Komplexbildner als Lösungsmittel sowohl bei der Katalysatorherstellung als auch bei der Hydroaminierungsreaktion zugegen sein.

So beschreiben z.B. J.F. Remenar (J. Am. Chem. Soc. 120 (1988), 4081ff), H. Lehmkuhl et al. (J. Organomet. Chem. 55 (1973), 215ff) und D. Steinborn et al. (Z. Chem. 29 (1989), 333ff) die Verwendung von N,N,N',N'-Tetramethylethylendiamin, N,N,N',N'',N''-Pentamethyldiethylentriamin, N,N,N',N'-Tetramethylcyclohexandiamin und Tetrahydrofuran als Komplexbildner.

Des weiteren können Amine mit mehreren aminischen N-Atomen pro Molekül, wie z.B. N,N,N',N'-Tetraethylethylendiamin, N-permethyliertes bzw. N-perethyliertes Triethylentetramin bis hin zu N-permethyliertem bzw. N-perethyliertem Polyimin mit Molmassen bis 500.000 Dalton, Ether und Polyether, wie z.B. Diglyme, Triglyme und die entsprechenden Homologen, endgruppenverschlossene Polyole - z.B. PEG, PPG, Poly-THF -, und Komplexbildner mit aminischen N- und etherischen O-Atomen im Molekül, wie z.B. 3-Methoxyethylamin, 3-(2-Methoxyethoxy)propylamin oder N,N,N',N'-Tetramethyldiaminodiethylether, zugegen sein.

Man geht dabei in der Regel so vor, dass man den Katalysator [oder das entsprechende Alkalimetall oder das entsprechende Metallhydrid oder die entsprechende Organometallverbindung (z.B. n-BuLi), jeweils als Katalysator'vorläufer'] in einem primären und/oder sekundären Amin, besonders bevorzugt in einem sekundären Amin, löst oder suspendiert.

Der Katalysator kann als Lösung, als Suspension oder geträgert auf einem typischen Katalysatorträger wie z.B. SiO₂, Al₂O₃, TiO₂, ZrO₂, Aktivkohle, MgO, MgAl₂O₄ vorliegen. Bevorzugt liegt der Katalysator als Lösung oder Suspension, besonders bevorzugt als Lösung vor.

Die Hydroaminierung des 1,3-Butadien-haltigen Gemischs kann diskontinuierlich, halbkontinuierlich oder kontinuierlich erfolgen.

Im ersten Fall wird das 1,3-Butadien-haltige Gemisch zu Katalysator und Amin gegeben und umgesetzt. Im zweiten Fall wird das 1,3-Butadien-haltige Gemisch zur Reaktionsmischung dosiert. Im dritten Fall werden Katalysator, Amin und 1,3-Butadien-haltige Gemisch kontinuierlich zudosiert.

Bevorzugt ist ein molares Verhältnis 1,3-Butadien-haltiges Gemisch : sekundärem Amin von 3:1 bis 1:10, besonders bevorzugt ist 1:1 bis 1:2.

Bevorzugt ist ein molares Verhältnis 1,3-Butadien-haltiges Gemisch : primärem Amin von 6:1 bis 1:5, besonders bevorzugt ist 2:1 bis 1:1.

Die Reaktion findet, bevorzugt unter Rühren, bei 0 bis 250°C, insbesondere bei 20 bis 150°C und besonders bevorzugt bei 40 bis 120°C statt.

Die Reaktion kann unter dem sich unter den gewählten Bedingungen ergebenden Druck (Eigendruck) durchgeführt werden. Im allgemeinen beträgt der Überdruck bei der Reaktionsdurchführung 0 bis 200 bar, insbesondere 2 bis 100 bar, ganz besonders 3 bis 30 bar.

Als Reaktoren kommen alle typischen Reaktionsapparate in Frage, z.B. Rührkessel, Schlaufenreaktoren, Blasensäulen, gepackte Blasensäulen, kaskadierte Blasensäulen und Rührkolonnen.

Nach der Reaktion wird das Produkt z.B. durch Destillation, Rektifikation, Filtration, Membranfiltration, Wasserwäsche oder Adsorption vom Katalysator getrennt.

Nichtprotolysierter Katalysator (Metallamid oder Metallhydrid) kann anschließend zurückgeführt werden.

### b) Übergangsmetallkomplexverbindung-Katalyse

Die Umsetzung des 1,3-Butadien-haltigen Gemischs mit dem primären und/oder dem sekundären Amin kann z.B. wie in der oben bei der Abhandlung der Übergangsmetallkomplexverbindungen genannten Literatur beschrieben erfolgen.

Die Reaktion kann kontinuierlich, in Batch-Fahrweise oder in Semi-Batch-Fahrweise betrieben werden.

Bei den Batch-Fahrweisen wird der Katalysator zusammen mit dem Amin vorgelegt. Nach Erreichen der Reaktionstemperatur wird das 1,3-Butadien-haltige Gemisch aufgepresst. Nach Absinken des Drucks (Maß für die Reaktion) wird das Produkt bzw. Produktgemisch abdestilliert.

Überschüssiges 1,3-Butadien sowie nicht abreagiertes Amin können zurückgeführt werden.

Bei der Batch-Fahrweise kann der Katalysator zusammen mit dem Produktgemisch über Sumpf aus dem Reaktor gefahren werden und separat aufgearbeitet werden.

Die Reaktion kann in einem Rührkessel durchgeführt werden.

Bei kontinuierlicher Fahrweise kann die Reaktion in einer Blasensäule durchgeführt werden. Hierbei wird das 1,3-Butadien-haltige Gemisch von unten durch das Gemisch aus Katalysator und Produktlösung geblasen. Die Lösung kann dann destillativ vom Katalysator entfernt oder der Katalysator kann mittels Membran aus der Produktlösung entfernt werden. Alternativ braucht der Katalystor nicht entfernt zu werden, sondern kann direkt in die Aufarbeitung bzw. in den nächsten Verfahrensschritt gegeben werden, sofern er dort nicht stört.

### Zweite Verfahrensstufe:

Im Anschluss an die Umsetzung des 1,3-Butadien-haltigen Gemischs mit dem primären und/oder sekundären Amin unter hydroaminierenden Bedingungen wird/werden das oder die erhaltene/n Hydroaminierungsprodukt/e in einer zweiten Verfahrensstufe unter umalkylierenden Bedingungen umgesetzt.

### Variante a)

Die Verfahrensprodukte der Umalkylierung in dieser zweiten Verfahrensstufe sind wiederum n-Butenylamine. Die Zusammensetzung des Reaktionsaustrags dieser Umalkylierungsstufe unterscheidet sich vor der der vorangegangenen Hydroaminierungsstufe u.a. dadurch, dass die als Produkte erhaltenen n-Butenylamine in einem anderen Molverhältnis zueinander vorliegen.

Diese zweite Verfahrensstufe wird bevorzugt in Gegenwart eines Umalkylierungskatalysators durchgeführt.

Bevorzugte Temperaturen bei dieser Amin-Umalkylierungsstufe sind 80 bis 400°C.

Der Reaktionsaustrag aus der ersten Hydroaminierungsstufe kann, in der Regel nach Abtrennung des Katalysators (wie jeweils oben beschrieben), direkt in diese zweite Verfahrensstufe eingesetzt werden.

Aus dem Reaktionsaustrag der ersten Hydroaminierungsstufe kann/können jedoch auch zunächst das oder die für diese zweite Verfahrensstufe gewünschte/n n-Butenylamine als Hydroaminierungsprodukt/e (wie jeweils oben beschrieben) abgetrennt und danach in diese zweite Verfahrensstufe eingesetzt werden.

Vorteilhafterweise können dem Zulauf für die zweite Verfahrensstufe auch Ammoniak und/oder Amine zugeführt, und/oder Ammoniak und/oder Amine aus dem Reaktionsaustrag dieser zweiten Verfahrensstufe zu(rück)geführt werden.

In einer bevorzugten Ausführungsform trennt man das in der zweiten Verfahrensstufe (Umalkylierungsstufe) erhaltene Produktgemisch auf in Produkt/e, das/die in die erste Verfahrensstufe (Hydroaminierungsstufe) zurückgeführt wird/werden, und/oder Produkt/e, das/die in die zweite Verfahrensstufe (Umalkylierungsstufe) zurückgeführt wird/werden, und das oder die gewünschte/n n-Butenylamin/e.

Die Umsetzung des/der in der ersten Verfahrensstufe erhaltenen Hydroaminierungsprodukts/-produkte in der zweiten Verfahrensstufe unter umalkylierenden Bedingungen kann z.B. wie in Houben Weyl Band XI/1, Stickstoffverbindungen II, 1957, Georg Thieme Verlag Stuttgart, S. 248 - 261, beschrieben erfolgen.

Demnach kann die Aminumalkylierung (,Amintausch') in Gegenwart von Umalkylierungskatalysatoren wie Säuren, Zeolithen, Metallsalzen, Iod, Dehydratationskatalysatoren, Hydrier-/Dehydrierkatalysatoren oder auch in Abwesenheit von Katalysatoren durchgeführt werden.

Als Umalkylierungskatalysator geeignete Säuren sind z.B. Halogenwasserstoffsäuren (wie HCl), Phosphorsäure, Sulfanilsäure oder Sulfonsäuren (wie p-Toluolsulfonsäure).

Als Umalkylierungskatalysator geeignete Zeolithe sind z.B. Faujasite wie X-, Y- und USY-Zeolith, Erionit, Chabazit, Mordenit, Offretit, Clinoptiolith, Pentasile wie ZSM-5 und ZBM-10, ZSM-11, ZSM-12, MCM-22, MCM-41, MCM-48, MCM-49, MCM-56, EMT, SSZ-26, SSZ-33, SSZ-37, CIT-1, PSH-3, NU-85, Beta sowie die borhaltigen Formen, wie z.B. ZBM-11, H-Bor-ZSM-5, H-Bor-Beta, H-Bor-ZSM-11, sowie die gallium- oder titanhaltigen Formen. Sie zeichnen sich durch eine hohe Anzahl an katalytisch aktiven Zentren aus, kombiniert mit einer großen Oberfläche.

Diese Zeolithe unterscheiden sich im Typ und in der Art der Nachbehandlung nach ihrer Herstellung (z.B. thermische Behandlung, Dealuminierung, Säurebehandlung, Metallioneneintausch, etc.).

Beispiele für geeignete Zeolithe finden sich in US-A-4,375,002, US-A-4,536,602, EP-A-305 564, EP-A-101 921 und DE-A-42 06 992.

Auch die aus EP-A-133 938, EP-A-431 451 und EP-A-132 736 bekannten Zeolithe, bei denen es sich um Bor-, Gallium-, Alumino- und Eisensilikatzeolithe handelt, die gegebenenfalls wie beschrieben mit Alkali-, Erdalkali- und Übergangsmetallen dotiert werden können, sind geeignet.

Weiterhin sind z.B. auch die aus CA-A-2 092 964 bekannten Beta-Zeolithe, die als kristalline Aluminosilikate bestimmter Zusammensetzung mit einer Porengröße von mehr als 5 Å definiert sind, geeignet.

Bevorzugt werden metall- oder halogenmodifizierte Beta-Zeolithe eingesetzt, wie z.B. in DE-A-195 30 177 beschrieben.

Insbesondere geeignet sind auch Zeolith-Katalysatoren des Pentasil-Typs mit einem SiO₂/Al₂O₃-Molverhältnis von größer/gleich 10, wie sie in EP-A-132 736 offenbart sind.

Als Umalkylierungskatalysator geeignete Metallsalze sind z.B. Zink- oder Eisenhalogenide, wie Zinkchlorid, Eisen(II)chlorid oder Eisen(III)chlorid.

Als Umalkylierungskatalysator geeignete Dehydratationskatalysatoren sind z.B. Mangan(II)oxid/Aktivkohle, Aluminiumsilikate, Al₂O₃, TiO₂ oder ZrO₂.

Als Umalkylierungskatalysator bevorzugt sind Hydrier- und Dehydrierkatalysatoren.

Dabei ist zur Aufrechterhaltung der katalytischen Aktivität die Anwesenheit von Wasserstoff vorteilhaft. Alternativ kann der Hydrier- oder Dehydrierkatalysator in regelmäßigen Abständen mit H₂ reduktiv von Belägen befreit werden.

Als Hydrier- und Dehydrierkatalysatoren sind Katalysatoren, die als katalytisch aktive Bestandteile Elemente, ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, jeweils in metallischer Form (Oxidationsstufe 0) oder in Form von Verbindungen wie z.B. Oxiden, die unter den Verfahrensbedingungen zum entsprechenden Metall reduziert werden, enthalten, besonders geeignet.

Die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram sind im allgemeinen insgesamt in Mengen von 0,1 bis 80 Gew.-%, bevorzugt 0,1 bis 70 Gew.-%, besonders bevorzugt 0,1 bis 60 Gew.-%, berechnet als Metall in der Oxidationsstufe 0, in der katalytisch aktiven Masse des Katalysators enthalten.

Bevorzugt sind Katalysatoren, die als katalytisch aktive Bestandteile Elemente, ausgewählt aus der Gruppe Kupfer, Silber, Cobalt, Nickel, Ruthenium, Rhodium, Palladium, Platin, Chrom und Molybdän, insbesondere ausgewählt aus der Gruppe Kupfer, Cobalt, Nikkel, jeweils in metallischer Form (Oxidationsstufe 0) oder in Form von Verbindungen wie z.B. Oxiden, die unter den Verfahrensbedingungen zum entsprechenden Metall reduziert werden, enthalten.

Mehr bevorzugt sind Katalysatoren, die die katalytisch aktiven Bestandteile Kupfer, Silber, Eisen, Cobalt, Nickel, Ruthenium, Rhodium, Palladium, Osmium, Iridium und/oder Platin und ein Trägermaterial, bevorzugt ausgewählt aus der Gruppe Aluminiumoxid, Zirkoniumdioxid, Titandioxid, Kohlenstoff und/oder sauerstoffhaltige Verbindungen des Siliziums, enthalten.

Die katalytisch aktive Masse dieser im erfindungsgemäßen Verfahren bevorzugt eingesetzten Katalysatoren enthält die katalytisch aktiven Bestandteile Kupfer, Silber, Eisen, Cobalt, Nickel, Ruthenium, Rhodium, Palladium, Osmium, Iridium und/oder Platin im allgemeinen insgesamt in Mengen von 0,1 bis 80 Gew.-%, bevorzugt 0,1 bis 70 Gew.-%, besonders bevorzugt 0,1 bis 60 Gew.-%, berechnet als Metall in der Oxidationsstufe 0.

Weiterhin enthält die katalytisch aktive Masse dieser bevorzugt eingesetzten Katalysatoren die Trägermaterialien Aluminiumoxid (Al₂O₃), Zirkoniumdioxid (ZrO₂), Titandioxid (TiO₂), Kohlenstoff und/oder sauerstoffhaltige Verbindungen des Siliziums, berechnet als SiO₂, im allgemeinen insgesamt in Mengen von 20 bis 99,9 Gew.-%, bevorzugt 30 bis 99,9 Gew.-%, besonders bevorzugt 40 bis 99,9 Gew.-%.

Besonders bevorzugt sind Katalysatoren mit den Aktivkomponenten Cu, Co, Ni und/oder Pd, insbesondere Cu, Co und/oder Ni. Diese können als Vollkontakte oder als Trägerkatalysatoren verwendet werden.

Ganz besonders bevorzugt werden Cu-haltige Katalysatoren, die, wie erfindungsgemäß erkannt wurde, wegen ihrer vergleichsweise geringen Ethan- bzw. Methanbildung selektiver sind.

Beispiele hierfür sind Kupferlegierungen, metallisches Kupfer, z.B. in Form von Kupfernetz, und Cu-Katalysatoren mit einem Cu-Gehalt von 2 bis 70 Gew.-% Cu, berechnet als CuO, auf einem Träger, bevorzugt mit 10 bis 55 Gew.-% Cu, berechnet als CuO, auf einem Träger. Trägermaterial können bevorzugt Aluminiumoxid (Al₂O₃), Zirkoniumdioxid (ZrO₂), Titandioxid (TiO₂), Kohlenstoff und/oder sauerstoffhaltige Verbindungen des Siliziums sein.

Beispielsweise können in der EP-A-382 049 offenbarte Katalysatoren, deren katalytisch aktive Masse vor der Behandlung mit Wasserstoff
20 bis 85 Gew.-%, bevorzugt 70 bis 80 Gew.-%, ZrO₂,
1 bis 30 Gew.-%, bevorzugt 1 bis 10 Gew.-%, CuO,
und jeweils 1 bis 40 Gew.-%, bevorzugt 5 bis 20 Gew.-%, CoO und NiO enthält, beispielsweise die in loc. cit. auf Seite 6 beschriebenen Katalysatoren mit der Zusammensetzung 76 Gew.-% Zr, berechnet als ZrO₂, 4 Gew.-% Cu, berechnet als CuO, 10 Gew.-% Co, berechnet als CoO, und 10 Gew.-% Ni, berechnet als NiO, im erfindungsgemäßen Verfahren eingesetzt werden.

Weiterhin können im erfindungsgemäßen Verfahren die in der EP-A-963 975 offenbarten Katalysatoren, deren katalytisch aktive Masse vor der Behandlung mit Wasserstoff
22 bis 40 Gew.-% ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO, eingesetzt werden.

Weiterhin können im erfindungsgemäßen Verfahren in der EP-A-514 692 offenbarte Katalysatoren, deren katalytisch aktive Masse vor der Behandlung mit Wasserstoff 5 bis 100 Gew.-% eines Oxides von Kupfer und Nickel im Atomverhältnis von 1 : 1 bis 10 : 1, bevorzugt von 2 : 1 bis 5 : 1, und Zirkon- und/oder Aluminiumoxid enthält, insbesondere die in loc. cit. auf Seite 3, Zeilen 20 bis 30, offenbarten Katalysatoren, deren katalytisch aktive Masse vor der Behandlung mit Wasserstoff 20 bis 80, besonders 40 bis 70, Gew.-% Al₂O₃ und/oder ZrO₂, 1 bis 30 Gew.-% CuO, 1 bis 30 Gew.-% NiO und 1 bis 30 Gew.-% CoO enthält, eingesetzt werden.

Bevorzugt können in der DE-A-19 53 263 offenbarte Katalysatoren enthaltend Kobalt, Nickel und Kupfer und Aluminiumoxid und/oder Siliciumdioxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt,
die in EP-A-696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃,
die in EP-A-284 919 offenbarten Katalysatoren der allgemeinen Formel MₓMg_{y}(SiO₂)•nH₂O, worin M ein zweiwertiges, reduzierbares Metallatom aus der Gruppe Cu, Fe, Co und Ni, x und y Zahlen sind, die zusammen den Wert 1,5 erreichen können, und n nach Trocknung ausgedrückt in Gew.-% zwischen 0 und 80 liegt, beispielsweise der in loc. cit im Beispiel beschriebene Katalysator enthaltend 35 % CuO, 9 % MgO und 38 % SiO₂ und der in EP-A-863 140 auf Seite 3 beschriebene Katalysator enthaltend 45 bis 47 Gew.-% CuO, Magnesiumsilikat aus etwa 15 bis 17 Gew.-% MgO und 35 bis 36 Gew.-% SiO₂, etwa 0,9 Gew.-% Cr₂O₃, etwa 1 Gew.-% BaO und etwa 0,6 Gew.-% ZnO,
die in DE-A-24 45 303 offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, bei einer Temperatur von 350 bis 700 °C erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird, und
die in WO 95/32171 und EP-A-816 350 offenbarten Trägerkatalysatoren enthaltend 5 bis 50, bevorzugt 15 bis 40, Gew.-% Kupfer, berechnet als CuO, 50 bis 95, bevorzugt 60 bis 85, Gew.-% Silicium, berechnet als SiO₂, 0 bis 20 Gew.-% Magnesium, berechnet als MgO, 0 bis 5 Gew.-% Barium, berechnet als BaO, 0 bis 5 Gew.-% Zink, berechnet als ZnO, und 0 bis 5 Gew.-% Chrom, berechnet als Cr₂O₃, jeweils bezogen auf das Gesamtgewicht des calcinierten Katalysators, beispielsweise der in EP-A-816 350, Seite 5, offenbarte Katalysator enthaltend 30 Gew.-% CuO und 70 Gew.-% SiO₂,
erfindungsgemäß eingesetzt werden.

Die im erfindungsgemäßen Verfahren als Umalkylierungskatalysator verwendeten Hydrier- oder Dehydrierkatalysatoren können nach den im Stand der Technik beschriebenen Verfahren hergestellt und teilweise auch kommerziell erhalten werden.

Bei der Herstellung von Trägerkatalysatoren existieren bezüglich der Aufbringungsmethode der Aktivkomponenten, wie z.B. Nickel, Cobalt und/oder Kupfer und gegebenenfalls weitere Komponenten, auf das verwendete Trägermaterial keinerlei Beschränkungen.

Insbesondere kommen als Aufbringungsmethoden die Tränkung und die Fällung, z.B. wie in der älteren deutschen Anmeldung Nr. 10041676.4 vom 24.08.00, Seiten 26 - 28, beschrieben, in Betracht.

### Variante b)

In einer bevorzugten Ausführungsform wird im Anschluss an die Umsetzung des 1,3-Butadien-haltigen Gemischs mit dem primären und/oder sekundären Amin unter hydroaminierenden Bedingungen das oder die erhaltene/n Hydroaminierungsprodukt/e in einer zweiten Verfahrensstufe unter umalkylierenden und hydrierenden Bedingungen umgesetzt.

Erfolgt die Umsetzung in der zweiten Verfahrensstufe unter umalkylierenden und gleichzeitig hydrierenden Bedingungen, insbesondere in Gegenwart von Wasserstoff und einem umalkylierenden Hydrier- oder Dehydrierkatalysator, können die im Hydroaminierungsaustrag aus der ersten Verfahrensstufe enthaltenen n-Butenylamine und gegebenenfalls weitere ungesättigte organische Sticktoffverbindungen, d. h. organische Stickstoffverbindungen, die entweder eine olefinische C=C-Doppelbindung (z.B. wie in Enaminen) aufweisen oder Imine darstellen, zu den entsprechend gesättigten n-Butylaminen hydriert werden.

Als umalkylierender Hydrier- oder Dehydrierkatalysator werden hierbei die gleichen Hydrier- und Dehydrierkatalysatoren wie bereits oben bei Variante a) beschrieben bevorzugt.

Der Hydroaminierungsaustrag aus der ersten Verfahrensstufe enthält z.B. neben den der n-Butenyl-Funktion in den n-Butenylaminen weitere ungesättigte Funktionen, wenn in der ersten Verfahrensstufe als Amin ein entsprechend ungesättigtes Amin eingesetzt wurde. Imine können durch Doppelbindungsisomerisierung oder durch β-Eliminierung aus Metallalkylamiden entstehen.

Beispiele für solche ungesättigte Amine mit einer olefinischen C=C-Doppelbindung sind: Allylamin, 1-Amino-2-buten, 1-Amino-3-buten, 1-Amino-4-penten.

Zur Durchführung der zweiten Verfahrensstufe in Gegenwart des Hydrier- oder Dehydrierkatalysators (Variante a) und b)) geht man bevorzugt so vor, dass das Produkt- bzw Produktgemisch aus der ersten Reaktionsstufe (Hydroaminierungsstufe), oder das aus der Aufarbeitung der ersten Verfahrensstufe (Hydroaminierungsstufe) stammende n-Butenylamin bzw. angereicherte n-Butenylamin kontinuierlich über den Umalkylierungskatalysator gefahren wird oder diskontinuierlich umalkyliert und gegebenenfalls hydriert wird.

Bei einer kontinuierlichen Fahrweise wird der Umalkylierungskatalysator in einen Rohrreaktor oder Rohrbündelreaktor eingebaut.

Im Falle eines umalkylierenden Dehydrier-/Hydrierreaktors und der Fahrweise in Gegenwart von H₂ (Variante b) kann der Katalysator wahlweise vorher mit Wasserstoff reduziert werden, er kann aber auch in Gegenwart des Produktes und Wasserstoff direkt angefahren werden.

Der Wasserstoffdruck (Variante b)) kann zwischen 0 bar und 300 bar, bevorzugt zwischen 1 und 250 bar, gewählt werden.

Bei einer Umsetzung in der Gasphase liegt der Druck im allgemeinen bei 1 bis 70 bar.

Bei einer Umsetzung in der Flüssigphase liegt der Druck im allgemeinen bei 70 bis 250 bar.

Die Temperatur (Variante a und b) liegt im allgemeinen bei 80 bis 400°C, insbesondere zwischen 100 und 350°C, bevorzugt zwischen 120 und 250°C, ganz besonders bevorzugt zwischen 150 und 230°C.

Je nach gewählter Temperatur stellt sich ein thermodynamisches Gleichgewicht der verfahrensgemäßen n-Butenyl- bzw. n-Butylamine plus ggf. Ammoniak ein, das von dem Verhältnis Stickstoff zu den Butyl- bzw. Butenylgruppen abhängt. Je sterisch anspruchsvoller die Amine sind, desto geringer wird der Anteil des entsprechenden tertiären n-Butenyl- bzw. n-Butylamins.

Die Belastung des Katalysators mit dem Startmaterial kann zwischen 0,05 und 2 kg Einsatzmaterial pro Liter Katalysator und pro Stunde (kg/l*h), bevorzugt zwischen 0,1 und 1 kg/l*h, besonders bevorzugt zwischen 0,2 und 0,6 kg/l*h, betragen.

Das molare Verhältnis der n-Butenyl- bzw. n-Butylamine (der ,Umalkylierungspartner') kann je nach gewünschtem Produktmix in weiten Bereichen variieren.

Im Falle einer diskontinuierlichen Umalkylierung kann der Katalysator zusammen mit dem Einsatzmaterial für die zweite Verfahrensstufe vorgelegt und die gewünschte Menge des Umalkylierungspartners dazugegeben oder ergänzt werden. Anschließend wird auf die gewünschte Temperatur erwärmt (s.o.).

Nach Druckentspannung kann der Austrag nach üblichen Methoden aufgearbeitet, z.B. aufdestilliert, werden.

In einer besonderen Verfahrensausgestaltung wird das in der zweiten Verfahrensstufe (Umalkylierung und Hydrierung) erhaltene Produktgemisch auftrennt in Produkt/e, das/die in die erste Verfahrensstufe (Hydroaminierung) zurückgeführt wird/werden, und/oder Produkt/e, das/die in die zweite Verfahrensstufe (Umalkylierung und Hydrierung) zurückgeführt wird/werden, und das oder die gewünschte/n n-Butylamin/e.

Erfindungsgemäß können auch n-Butylamine hergestellt werden, indem man zunächst wie bereits beschrieben durch Umsetzung von 1,3-Butadien in Form eines 1,3-Butadien-haltigen Gemischs, das man bei der Spaltung von Erdölfraktionen oder bei der Dehydrierung von LPG oder LNG oder aus der GTL-Technologie erhält, mit einem primären Amin und/oder einem sekundären Amin unter hydroaminierenden Bedingungen und anschließende Umsetzung des/der erhaltenen Hydroaminierungsprodukte/s in einer zweiten Verfahrensstufe unter umalkylierenden Bedingungen n-Butenylamine herstellt und in einer weiteren Verfahrensstufe unter hydrierenden Bedingungen umsetzt.

Diese weitere Verfahrensstufe unter hydrierenden Bedingungen kann z.B. wie bereits oben beschrieben durch entsprechende Umsetzung des oder der n-Butenylamins/e in Gegenwart eines Hydrier- oder Dehydrierkatalysators und Wasserstoff durchgeführt werden.

Die Reste R¹ bis R⁵ in den Formeln II und III bedeuten unabhängig voneinander:
R¹, R³, R⁴:
   Wasserstoff (H),
R¹, R², R⁴, R⁵:
   Alkyl, insbesondere C₁- bis C₂₀-Alkyl, bevorzugt C₁- bis C₁₂-Alkyl, besonders bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, neo-Pentyl, n-Hexyl, iso-Hexyl, n-Heptyl, iso-Heptyl, n-Octyl und iso-Octyl,
   Alkenyl, insbesondere C₂- bis C₂₀-Alkenyl, bevorzugt C₂- bis C₁₂-Alkenyl, besonders bevorzugt C₂- bis C₈-Alkenyl, wie Vinyl und Allyl,
   Cycloalkyl, insbesondere C₃- bis C₂₀-Cycloalkyl, bevorzugt C₃- bis C₁₂-Cycloalkyl, besonders bevorzugt C₅- bis C₈-Cycloalkyl, wie Cyclopentyl, Cyclohexyl, Cycloheptyl und Cyclooctyl,
   Alkoxyalkyl, insbesondere C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, besonders bevorzugt C₂- bis C₄-Alkoxyalkyl,
   Aminoalkyl, insbesondere C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Aminon-pentyl, N-(2-Aminoethyl)-2-aminoethyl und N-(2-Aminoethyl)aminomethyl,
   Monoalkylaminoalkyl, insbesondere C₂₋₃₀-Monoalkylaminoalkyl, bevorzugt C₂₋₂₀-Monoalkylaminoalkyl, besonders bevorzugt C₂₋₈-Monoalkylaminoalkyl, wie Methylaminomethyl, 2-Methylaminoethyl, Ethylaminomethyl, 2-Ethylaminoethyl und 2-iso-Propylaminoethyl,
   Dialkylaminoalkyl, insbesondere C₃₋₃₀-Dialkylaminoalkyl, bevorzugt C₃₋₂₀-Dialkylaminoalkyl, besonders bevorzugt C₃₋₁₀-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Diiso-propylamino)ethyl,
   Aryl, wie Phenyl, 1-Naphthyl und 2-Naphthyl, bevorzugt Phenyl,
   Aralkyl, insbesondere C₇- bis C₂₀-Aralkyl, bevorzugt C₇- bis C₂₀-Aralkyl, bevorzugt C₇- bis C₁₂-Phenalkyl, wie Phenylmethyl, 1-Phenylethyl, 2-Phenylethyl,
R¹ und R², R⁴ und R⁵:
   Gemeinsam eine gesättigte oder ungesättigte C₃- bis C₉-Alkylenkette, die durch ein O-, S- oder N-Heteroatom unterbrochen sein kann, wie z.B. -(CH₂)₃-, -(CH₂)₄-, -(CH₂)₅-, -(CH₂)₆-, -(CH₂)₇- und -CH=CH-CH=CH-,
   insbesondere gemeinsam eine -(CH₂)ⱼ-X-(CH₂)ₖ- Gruppe, mit j und k unabhängig voneinander = 1, 2, 3 oder 4 und X = CH₂, CHR³, Sauerstoff (O), Schwefel (S) oder NR³,
wobei R³ = H oder C₁- bis C₄-Alkyl, wie Methyl, Ethyl, n-Propyl, iso-Propyl, n-Butyl, iso-Butyl, sec.-Butyl, tert.-Butyl,
wie z.B. -(CH₂)₂-NH-(CH₂)₂-, -(CH₂)₂-NCH₃-(CH₂)₂-, -(CH₂)₂-O-(CH₂)₂-.

### Beispiele

### Beispiel 1 (Hydroaminierung von 1,3-Butadien in Form eines 1,3-Butadien-haltigen C4-Schnitts mit Di-n-butylamin):

50 mmol Na wurden in 30 ml wasserfreiem n-Octan bei 110°C geschmolzen und durch Rühren dispergiert. Nach dem Abkühlen wurden 258 g über 3Å-Molsieb getrocknetes Di-n-butylamin in einen 1 1-Reaktor gegeben, die Na-Suspension unter Ausschluss von Sauerstoff und Wasser zugegeben und unter Rühren innerhalb von 2 h C4-Schnitt (235 g) mit der Zusammensetzung (in Gew.-%)

| | |
|---|---|
| 1,3-Butadien | 42,3 |
| Butene | 47,5 |
| Butane | 8,7 |
| Alkine | 0,8 |
| Sonstige | 0,7 |

über ein Nadelventil in den Reaktor (1 l) gefahren.

| Zeit [Min.] | Druck [bar] | C4-Schnitt in g | Temp. [°C] |
|---|---|---|---|
| 0 | 0 | 0 | 58 |
| 5 | 0,5 | 0,5 | 58 |
| 15 | 1,4 | 16 | 58 |
| 30 | 4 | 44 | 58 |
| 60 | 9 | 120 | 59 |
| 90 | 17 | 195 | 60 |
| 110 | 23 | 235,4 | 57 |
| 135 | 20 | | 61 |
| 180 | 20 | | 62 |

Anschließend wurde auf 50°C abgekühlt und der Gasraum in ein mit Trockeneis/Aceton gekühltes Kondensationsgefäß entspannt. Danach wurden 10 ml 50 %ige wässrige KOH in den Rührautoklav gegeben (GC-Analyse: Rtx5-Amine, 100°C - 5°C/Min. - 230°C).

Flüssiger Reaktorinhalt: 344,8 g bestehend aus:

| Substanz | in g | in % |
|---|---|---|
| Di-n-butylamin | 61,9 | 25.0 |
| Di-n-butyl-n-butenylamine | 271,1 | 74.3 |
| Sonstige | | 0,7 |

Kondensat: 158 g bestehend aus:

| GC-Analyse | vorher | nachher |
|---|---|---|
| [Flächen %] | | |
| i-Butan | 1,78 | 2,67 |
| n-Butan | 6,02 | 9,41 |
| 2-Buten-trans | 4,82 | 8,38 |
| Buten-1 | 13,80 | 18,5 |
| i-Buten | 24,4 | 38,9 |
| 2-Buten-cis | 3,61 | 8,38 |
| i-Pentan | 0,040 | 0,041 |
| n-Pentan | 0,027 | 0,02 |
| 1,3-Butadien | 43,7 | 14,6 |
| Butenin | 0,586 | 0 |
| 1-Butin | 0,126 | 0 |
| Unbekannte (C5+) | 1,09 | 0,32 |

### Beispiel 2 (Hydrierung des in Beispiel 1 gewonnenen, Di-n-butyl-n-butenylamin-haltigen Reaktionsaustrags):

In einem 200 ml Autoklaven wurden 40 g des schwach gelben Reaktionsaustrages aus Beispiel 1 an einem Ni/Co/Cu/Al₂O₃-Hydrierkontakt (10 Gew.-% CoO, 10 Gew.-% NiO, 4 Gew.-% CuO auf Aluminiumoxid; hergestellt gemäß DE-A-19 53 263) bei 150°C und 200 bar Wasserstoff 4 h hydriert. Der Austrag (40,2 g, wasserklar) enthielt 74,1 % Tri-n-butylamin und 25,1 g Di-n-butylamin.

### Beispiel 3 (Hydrierende Umalkylierung des in Beispiel 1 gewonnenen, Di-n-butyl-n-butenylamin-haltigen Reaktionsaustrags):

In einem 200 ml Autoklaven wurden 20 g des Reaktionsaustrages aus Beispiel 1 an einem Ni/Co/Cu/Al₂O₃-Hydrierkontakt (10 Gew.-% CoO, 10 Gew.-% NiO, 4 Gew.-% CuO auf Aluminiumoxid; hergestellt gemäß DE-A-19 53 263) bei 190°C mit 40 ml Ammoniak beschickt. Anschließend wurde Wasserstoff auf 200 bar aufgepresst und 4 h bei 190°C gehalten. Nach Abkühlen und langsamem Entspannen auf Normaldruck erhielt man einen Austrag (22,1 g, wasserklar), der 15,2 % Mono-n-butylamin, 52,8 % Di-n-butylamin, 31,8 % Tri-n-butylamin und 0,2 % Nebenprodukte enthielt (Angaben in GC-Flächen%).

## Patentansprüche

1. Verfahren zur Herstellung eines 1,3-Butadien-freien Gemischs aus einem entsprechenden 1,3-Butadien-haltigen Gemisch, **dadurch gekennzeichnet, dass** man 1,3-Butadien in Form eines 1,3-Butadien-haltigen Gemischs, das man bei der Spaltung von Erdölfraktionen oder bei der Dehydrierung von LPG oder LNG oder aus der GTL-Technologie erhält, mit einem primären Amin und/oder einem sekundären Amin unter hydroaminierenden Bedingungen umsetzt, wobei man die Hydroaminierung in Gegenwart eines Alkalimetalls als Katalysator oder die Hydroaminierung in Gegenwart eines Metallmonoalkylamids oder Metalldialkylamids oder eines dem eingesetzten Amin entsprechenden Metallamids als Katalysator durchführt, und anschließend das oder die erhaltene/n Hydroaminierungsprodukt/e in einer zweiten Verfahrensstufe unter umalkylierenden Bedingungen umsetzt.

2. Verfahren nach Anspruch 1, **dadurch gekennzeichnet, dass** es sich bei dem 1,3-Butadien-haltigen Gemisch um einen C4-Schnitt handelt.

3. Verfahren nach einem der vorhergehenden Ansprüche, **dadurch gekennzeichnet, dass** das 1,3-Butadien-haltige Gemisch einen Gehalt an 1,3-Butadien von 5 bis 50 Gew.-% aufweist.

4. Verfahren nach einem der vorhergehenden Ansprüche, **dadurch gekennzeichnet, dass** das 1,3-Butadien-haltige Gemisch weniger als 2 Gew.-% Kohlenwasserstoffe enthält, die nach der MSAD-Skala einen pKs-Wert kleiner 30 besitzen.

5. Verfahren nach einem der Ansprüche 1 bis 4, **dadurch gekennzeichnet, dass** man die Hydroaminierung in Gegenwart von Natrium als Katalysator durchführt.

6. Verfahren nach einem der Ansprüche 1 bis 4, **dadurch gekennzeichnet, dass** man die Hydroaminierung in Gegenwart eines Alkalimetalldialkylamids als Katalysator durchführt.

7. Verfahren nach einem der Ansprüche 1 bis 4, **dadurch gekennzeichnet, dass** man die Hydroaminierung in Gegenwart eines Natriumdialkylamids als Katalysator durchführt.

8. Verfahren nach einem der vorhergehenden Ansprüche, **dadurch gekennzeichnet, dass** man die Umsetzung in der zweiten Verfahrensstufe in Gegenwart von Ammoniak durchführt.

9. Verfahren nach einem der vorhergehenden Ansprüche, **dadurch gekennzeichnet, dass** man die Umsetzung in der zweiten Verfahrensstufe bei Temperaturen von 80 bis 400 °C durchführt.

10. Verfahren nach einem der vorhergehenden Ansprüche, **dadurch gekennzeichnet, dass** man die Umsetzung in der zweiten Verfahrensstufe in Gegenwart eines Umalkylierungskatalysators durchführt.

11. Verfahren nach einem der vorhergehenden Ansprüche, **dadurch gekennzeichnet, dass** man die Umsetzung in der zweiten Verfahrensstufe in Gegenwart eines umalkylierenden Hydrier- oder Dehydrierkatalysators durchführt.

12. Verfahren nach einem der vorhergehenden Ansprüche, **dadurch gekennzeichnet, dass** man das in der zweiten Verfahrensstufe erhaltene Produktgemisch auftrennt in Produkt/e, das/die in die erste Verfahrensstufe zurückgeführt wird/werden, und/oder Produkt/e, das/die in die zweite Verfahrensstufe zurückgeführt wird/werden, und n-Butenylamin/e.

13. Verfahren nach einem der vorhergehenden Ansprüche, **dadurch gekennzeichnet, dass** man die nach Durchführung der Umsetzung verbleibenden Bestandteile des eingesetzten 1,3-Butadien-haltigen Gemischs als Raffinat I weiterverarbeitet.

14. Verfahren nach einem der vorhergehenden Ansprüche, **dadurch gekennzeichnet, dass** man n-Butylamin als primäres Amin einsetzt.

15. Verfahren nach einem der vorhergehenden Ansprüche, **dadurch gekennzeichnet, dass** man Di-n-butylamin als sekundäres Amin einsetzt.
